# EUROPEAN PATENT SPECIFICATION

(11) **EP 0 535 801 B1**
(45) Date of publication and mention of the grant of the patent: **28.02.1996**
(21) Application number: 92307778.8
(22) Date of filing: 26.08.1992
(51) Int. Cl.: A61F 5/441, A61F 5/445

(54) **Medico-surgical collection bags**
Medizinischer chirurgischer Beutel
Sac collecteur médico-chirurgical

(30) Priority: 06.09.1991 GB 9119064
(43) Date of publication of application: 07.04.1993
(73) Proprietor: Smiths Industries Public Limited Company, London, NW11 8DS (GB)
(72) Inventor: Cross, David Edward, Rustington, West Sussex (GB); Brooks, Kenneth John, Lancing, West Sussex BN15 9JY (GB)
(74) Representative: Flint, Jonathan McNeill

(56) References cited:
- EP-A- 0 089 110
- EP-A- 0 130 019
- EP-A- 0 142 262
- EP-A- 0 156 164
- BE-A- 804 946
- GB-A- 2 185 404
- US-A- 3 902 496
- US-A- 4 938 749
- US-A- 4 938 750

## Description

This invention relates to medico-surgical collection bags for body waste products of the kind comprising a front wall and a rear wall of flexible material, the rear wall having an opening into the bag by which waste material can enter the bag, means for securing the bag to the wearer, and a vent in one of the walls.

The invention is more particularly concerned with ostomy bags and their methods of use.

Ostomy bags, such as described in GB-A-2185404, are used to collect faecal matter from a surgically-made stoma where a part of the patient's bowel is brought to the skin of the abdomen. The bags are usually of flexible plastics material and are attached to the patient by means of an adhesive, which may be on the bag itself around its opening, or on a separate patient fitment to which the bag can be coupled and uncoupled. It is common practice to provide the bag with a vent to allow for the escape of flatus gas. A filter, such as incorporating activated charcoal, may be included in the vent to reduce odour from vented gas.

One problem experienced with ostomy bags is referred to as "pancaking". This is where faecal matter, instead of dropping through the bag opening to the bottom of the bag, collects on the front wall of the bag opposite the opening. The collection of faecal material in this region obstructs the opening into the bag and can lead to escape of material between the seal of either the bag to the skin, or the bag to the patient fitment, or the patient fitment to the skin. It can also, in some cases, lead to detachment of the bag. The risk of pancaking is aggravated when the front wall lies close to the opening. Because the bags are supplied flat, it is usual for the front and rear walls to be in contact or close proximity when removed from their packaging. The action of attaching the bag to the wearer may, in some cases, involve pressing the front wall rearwardly towards the skin so as to bring the adhesive on the rear wall of the bag into contact with the skin or patient fitment. If the bag has a vent, it is possible, after attachment of the bag, for the front wall of the bag to be gripped and pulled forwardly away from the rear wall and the opening, the vent allowing air to enter the bag as this is done. Although this may reduce the risk of pancaking initially, it can be seen that, as air in the bag escapes through the vent (such as caused by external pressure from clothing), the front wall can move rearwardly towards the opening and thereby increase the risk of pancaking. If the bag has no vent, it will not be possible to pull the front wall forwardly because of the vacuum this creates.

Another problem with vented bags relates to the mounting of the filter on the vent because this must allow for efficient filtering whilst avoiding expensive assembly operations.

It is an object of the present invention in its various aspects to provide a medico-surgical collection bag that can be used to avoid these problems.

According to one aspect of the present invention there is provided a medico-surgical collection bag of the above-specified kind, characterised in that the vent includes two vent passages one of which is filtered and the other of which is unfiltered and a seal for sealing the vent such that both passages can be closed or either passage opened so that when the unfiltered passage is open, the front wall can be pulled away from the rear wall and when both passages are closed, gas is trapped within the bag.

The vent may include an aperture in the one wall, the filter being of disc shape with lateral dimensions smaller than the aperture, a gas-impermeable member being secured to the outer surface of the one wall around the aperture and to the outer surface of the filter such as to support it within the aperture, and the gas-impermeable member having an opening therethrough overlying the filter such that when the vent is open gas can escape from the bag through the filter and the opening. The inner surface of the filter may be gas impermeable so that gas flow is through the edge of the filter radially to the opening in the gas-impermeable member. The seal may include a flexible adhesive strip. The two vent passages may open at locations spaced from one another such that either of the vent passages can be exposed by peeling back the flexible adhesive strip.

According to another aspect of the present invention there is provided a method of use of a medico-surgical collection bag of the kind comprising a front wall and a rear wall of flexible material, the rear wall having an opening into the bag by which waste material can enter the bag, means for securing the bag to the wearer, and a vent in one of the walls having both a filtered passage and an unfiltered passage, the method including the steps of opening the unfiltered passage, pulling the walls apart so that air enters the bag through the unfiltered passage and subsequently sealing the vent to trap air within the bag and thereby keep the two walls separated from one another in the region of the opening.

A two-part ostomy ostomy bag assembly and its method of use, in accordance with the present invention, will now be described, by way of example, with reference to the accompanying drawings, in which:

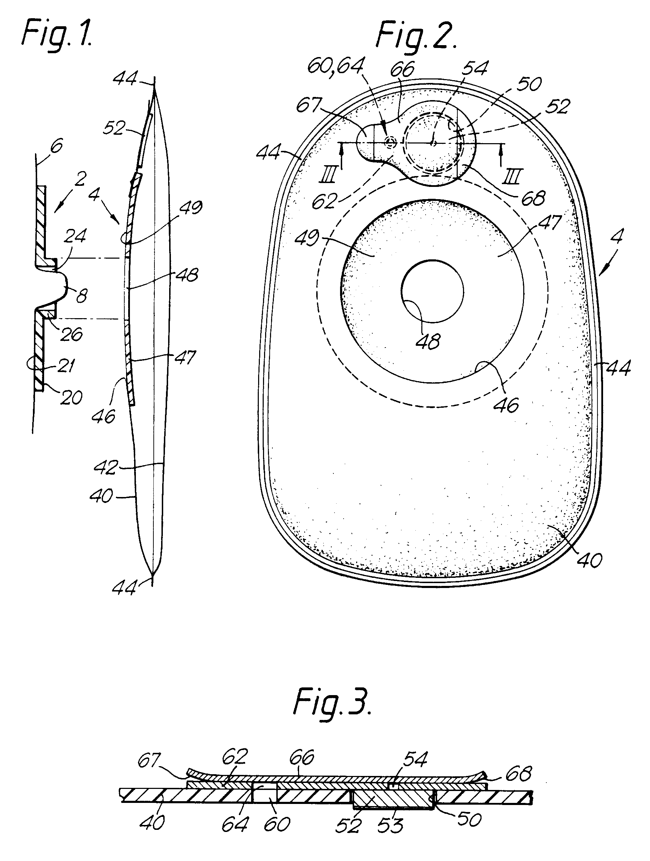

- Figure 1: is a sectional side elevation view of the assembly;
- Figure 2: is a view of the rear side of the bag; and
- Figure 3: is a transverse section to an enlarged scale along the line III - III of Figure 2.

The ostomy bag assembly is of the two-part kind, comprising a patient fitment 2 and a collection bag 4. The patient fitment 2 consists of a bendable plastics disc 20 with an adhesive rear surface 21 that is, in use, secured to the wearer's skin 6 around a stoma 8 which projects through a central aperture 24 in the fitment. A shallow collar 26 projects around the aperture 24 for use in locating with the bag 4.

The bag 4 comprises a rear wall 40 and a front wall 42 of a flexible plastics material, which may be w.c. disposable, sealed together around their outer edge 44. The front wall 42 is uninterrupted and imperforate, and is presented outwardly, away from the wearer's skin . The rear wall 40 has an opening 46 of diameter about 75mm and a flexible flange 47 with a diameter of about 95mm which has an adhesive rear surface that is stuck to the inside of the wall 40 around the opening 46. The flange 47 has a central aperture 48 of diameter about 25mm which provides the opening through which material discharged from the stoma 8 can enter the bag 4. That part of the rear surface of the flange 47 whch is exposed within the aperture 46 in the wall 40 provides an annular adhesive region 49 by which the bag can be secured to the front surface of the patient fitment 2.

Located directly above the opening 46 there is a first circular vent aperture 50 in the rear wall 40 which is about 25mm in diameter. A circular disc filter 52 of activated carbon fabric, about 23mm in diameter is located within the first vent aperture 50. The inner face of the filter 52 has a layer 53 of a gas-impermeable material bonded to it whereas the outer face of the filter is adhered to a strip of transparent tape 62. The tape 62 covers the filter 52 and, where it overlaps the filter, adheres to the outer surface of the rear wall 40. The tape 62 has an opening 54 about 2mm in diameter located centrally of the filter 52. Because the layer 53 prevents gas flow through the inner face of the filter 52, flow is radially-inwardly through the edge of the filter and out through the opening 54 in the tape 62. In this way, a long gas path through the filter is produced with the most efficient filtering. Flow into the filter 52 at its edge is facililated by the clearance between the edge of the filter and the aperture 50. Mounting the filter in this way can be accomplished readily by automated assembly operations whilst also giving effective filtering.

In alternative arrangements, the filter disc could be located internally or externally of the bag.

The bag 4 also has a second venting passage provided by an unfiltered vent aperture 60 which is located about 18mm to the left of the filtered vent aperture 50. The second vent aperture 60 is about 3mm in diameter and opens directly into the bag. The tape 62 also overlies the region around the aperture 60 and has an opening 64 in alignment with the vent aperture 60.

The region around the two apertures 50 and 60 is protected by the tape 62 adhered to the outer surface of the rear wall 40. The tape 62 is self-wound, single-sided, pressure-sensitive adhesive tape such as that sold by 3M under code 8415.

The bag 4 is completed by a vent sealing tape 66 which adheres to the rear face of tape 62 to extend over both the apertures 50 and 60 and thereby prevent flow of gas therethrough. The tape 66 is of the same material and size as the tape 62 and is located in register with the underlying tape 62, with the adhesive surface of the sealing tape in contact with the non-adhesive surface of the underlying tape. At opposite ends of the sealing tape 66 there are two loose peel tabs 67 and 68 formed by paper slips adhered to the adhesive side of the tape. In this way, either tab 67 or 68 can be grasped and the tape 66 peeled off. This does not disturb the underlying tape 62 because the force required to remove the sealing tape 66 in the peel mode is less than that required to remove the underlying tape in the shear mode.

Although it might be possible to seal the vents 50 and 60 by the sealing tape 66 alone, without the underlying tape 62, it has been found that the action of unpeeling the sealing tape can stretch the wall 40 of the bag and lead to wrinkling. This can make it difficult to reapply the sealing tape 66 in a way that provides an effective seal.

The assembly is used in the following way. The patient fitment 2 is adhered to the skin around the stoma in the usual way. The bag 4 is held up to the patient fitment 2, with both vent apertures 50 and 60 closed by the sealing tape 66, so that the adhesive region 48 around the bag opening 46 adheres to the front surface of the patient fitment. To produce an effective seal, the user may have to press the front wall 42 firmly against the adhesive region 49 so that this is pressed into contact with the patient fitment 2; this has the effect of expelling most of the residual air in the bag 4 around the join with the patient fitment 2 before the seal is completed.

Once the bag 4 is securely attached to the patient fitment 2, the user can, if he wishes, admit air to the bag in order to reduce the risk of pancaking. This is done by peeling the sealing tape 66 back so that the unfiltered vent aperture 60 is exposed. The user then grips the front wall 42 and pulls it forwardly away from the rear wall 40. This creates a reduced pressure within the bag 4 which sucks in air through the vent aperture 60. The user then smooths the sealing tape 66 back over the aperture 60 so that the air cannot escape from the bag. In this way, the trapped air keeps the front wall 42 away from the rear wall 40, so that the risk of faeces collecting on the rear wall where they enter the bag is considerably reduced.

If gas should build up in the bag 4, this can be released by the user pealing back the sealing tape 66 to expose either the filtered opening 54 or the unfiltered opening 60, as desired. The gas is allowed to escape until the bag 4 reaches its desired inflation level and the sealing tape 66 is replaced.

If the user does not find pancaking to be a problem, he can permanently expose the filtered vent by peeling back the sealing tape 66 and folding a part of the adhesive surface onto itself so that the tape remains adhered to the bag, sealing the unfiltered vent.

It will be appreciated that the invention in its various aspects could be used with other two-part assemblies, such as that described in EP 476847, but is not confined to two-part bag assemblies and could be used with bags that are adhered directly to the user's skin.

## Claims

1. A medico-surgical collection bag for body waste products comprising a front wall (42) and a rear wall (40) of flexible material, the rear wall (40) having an opening (48) into the bag by which waste material can enter the bag, means (2, 47) for securing the bag to the wearer, and a vent (60, 64, 50, 52, 54, 66) in one of the walls, characterised in that the vent (60, 64, 50, 52, 54, 66) includes two vent passages (50, 54 and 60, 64) one of which (50, 54) is filtered and the other of which (60, 64) is unfiltered and a seal (66) for sealing the vent (60, 64, 50, 52, 54, 66) such that both passages (50, 54 and 60, 64) can be closed or either passage opened so that when the unfiltered passage (60, 64) is open, the front wall (42) can be pulled away from the rear wall (40) and when both passages are closed, gas is trapped within the bag.

2. A medico-surgical collection bag according to Claim 1, characterised in that the vent (60, 64, 50, 52, 54, 66) includes an aperture (50) in the one wall (40), that the filter (52) is of disc shape with lateral dimensions smaller than the aperture, that a gas-impermeable member (62) is secured to the outer surface of the one wall around the aperture (50) and to the outer surface of the filter (52) such as to support it within the aperture, and that the gas-impermeable member (62) has an opening (54) therethrough overlying the filter (52) such that when the vent is open gas can escape from the bag through the filter and the opening.

3. A medico-surgical collection bag according to Claim 2, characterised in that the inner surface (53) of the filter (52) is gas impermeable so that gas flow is through the edge of the filter radially to the opening (54) in the gas-impermeable member (62).

4. A medico-surgical collection bag according to any one of the preceding claims, characterised in that the seal includes a flexible adhesive strip (66).

5. A medico-surgical collection bag according to Claim 4, characterised in that the two vent passages (50, 54 and 60, 64) open at locations spaced from one another such that either of the vent passages can be exposed by peeling back the flexible adhesive strip (66).

6. A method of use of a medico-surgical collection bag of the kind comprising a front wall (42) and a rear wall (40) of flexible material, the rear wall having an opening (48) into the bag by which waste material can enter the bag, means (2, 47) for securing the bag to the wearer, and a vent (60, 64, 50, 52, 54, 66) in one of the walls having both a filtered passage (50, 54) and an unfiltered passage (60, 64), the method including the steps of opening the unfiltered passage (60, 64), pulling the walls (40 and 42) apart so that air enters the bag through the unfiltered passage and subsequently sealing the vent (60, 64, 50, 52, 54, 66) to trap air within the bag and thereby keep the two walls separated from one another in the region of the opening (49).

## Patentansprüche

1. Medizinisch-chirurgischer Sammelbeutel für Körperausscheidungsprodukte, bestehend aus einer Vorderwand (42) und einer Rückwand (40) aus flexiblem Material, wobei die Rückwand (40) eine Öffnung (48) in den Beutel aufweist, durch welche das ausgeschiedene Material in den Beutel gelangen kann, Mittel (2, 47) zum Befestigen des Beutels am Träger und eine Entlüftung (60, 64, 50, 52, 54, 66) in einer der Wände, **dadurch gekennzeichnet**, daß die Entlüftung (60, 64, 50, 52, 54, 66) zwei Entlüftungsdurchlässe (50, 54 und 60, 64) umfaßt, von denen eine (50, 54) gefiltert und die andere (60, 64) ungefiltert ist und eine Abdichtung (66) zum Abdichten der Entlüftung (60, 64, 50, 52, 54, 66), so daß beide Durchlässe (50, 54 und 60, 64) geschlossen und ein Durchlaß geöffnet werden kann, so daß wenn der ungefilterte Durchlaß (60, 64) offen ist, die Vorderwand (42) von der Rückwand (40) weggezogen werden kann und wenn beide Durchlässe geschlossen sind, Gas im Beutel eingeschlossen ist.

2. Medizinisch-chirurgischer Sammelbeutel nach Anspruch 1, **dadurch gekennzeichnet**, daß die Entlüftung (60, 64, 50, 52, 54, 66) eine Öffnung (50) in einer Wand (40) umfaßt, daß das Filter (52) scheibenförmig ausgebildet ist, mit Seitenabmessungen kleiner als die Öffnung, daß ein gasundurchlässiges Teil (62) an der äußeren Oberfläche der einen Wand um die Öffnung (50) herum und der äußeren Oberfläche des Filters (52) befestigt ist, derart, daß es innerhalb der Öffnung gehalten wird und daß das gasundurchlässige Teil (62) eine Öffnung (54) aufweist, welche über dem Filter (52) liegt, so daß wenn die Entlüftung offen ist, Gas vom Beutel durch das Filter und die Öffnung entweichen kann.

3. Medizinisch-chirurgischer Sammelbeutel nach Anspruch 2, **dadurch gekennzeichnet**, daß die innere Oberfläche (53) des Filters (52) gasundurchlässig ist, so daß ein Gasfluß durch die Kante des Filters radial zur Öffnung (54) im gasundurchlässigen Teil (62) erfolgt.

4. Medizinisch-chirurgischer Sammelbeutel nach einem der vorhergehenden Ansprüche, **dadurch gekennzeichnet**, daß die Abdichtung einen flexiblen Klebestreifen (66) umfaßt.

5. Medizinisch-chirurgischer Sammelbeutel nach Anspruch 4, **dadurch gekennzeichnet**, daß die beiden Entlüftungsdurchlässe (50, 54 und 60, 64) an Stellen sich öffnen, welche voneinander entfernt sind und daß eine der Entlüftungsdurchlässe freigelegt werden kann durch Abziehen des flexiblen Klebestreifens (66).

6. Verfahren zur Verwendung eines medizinisch-chirurgischen Sammelbeutels der Art, eine Vorderwand (42) und eine Rückwand (40) aus flexiblem Material umfassend, wobei die Rückwand eine Öffnung (48) in den Beutel aufweist, durch welche ausgeschiedenes Material in den Beutel eintreten kann, Mittel (2, 47) zur Befestigung des Beutels am Träger und eine Entlüftung (60, 64, 50, 52, 54, 66) in einer der Wände, welche einen gefilterten Durchlaß (50, 54) und einen ungefilterten Durchlaß (60, 64) aufweist, wobei das Verfahren die Schritte des Öffnens des ungefilterten Durchlasses (60, 64), des Auseinanderziehens der Wände (40 und 42), so daß Luft in den Beutel durch den ungefilterten Durchlaß und das darauffolgende Abdichten der Entlüftung (60, 64, 50, 52, 54, 66) umfaßt, um Luft innerhalb des Beutels einzuschließen und hierdurch im Bereich der Öffnung (49) die Wände voneinander zu trennen.

## Revendications

1. Sac collecteur médico-chirurgical pour déjections corporelles comportant une paroi avant (42) et une paroi arrière (40) en un matériau flexible, la paroi arrière (40) ayant une ouverture (48) dans le sac par laquelle les déjections peuvent entrer dans le sac, des moyens (2, 47) pour fixer le sac au porteur, et un évent (60, 64, 50, 52, 54, 66) dans l'une des parois, caractérisé en ce que l'évent (60, 64, 50, 52, 54, 66) comporte deux passages d'évent (50, 54 et 60, 64) dont l'un (50, 54) est filtré et dont l'autre (60, 64) n'est pas filtré, et un joint (66) pour sceller l'évent (60, 64, 50, 52, 54, 66) de sorte que les deux passages (50, 54 et 60, 64) peuvent être fermés ou chacun des passages peut être ouvert afin que, lorsque le passage non filtré (60, 64) est ouvert, la paroi avant (42) peut être détachée de la paroi arrière (40) et lorsque les deux passages sont fermés, le gaz est retenu dans le sac.

2. Sac collecteur médico-chirurgical selon la revendication 1, caractérisé en ce que l'évent (60, 64, 50, 52, 54, 66) comporte une ouverture (50) dans l'une des parois (40), en ce que le filtre (52) a une forme de disque dont les dimensions latérales sont inférieures à l'ouverture, en ce qu'un élément (62) imperméable aux gaz est fixé à la surface extérieure de l'une des parois autour de l'ouverture (50) et à la surface extérieure du filtre (52) de façon à le soutenir dans l'ouverture, et en ce que l'élément (62) imperméable aux gaz a une ouverture (54) traversante surmontant le filtre (52) afin que, lorsque l'évent est ouvert, du gaz peut s'échapper du sac à travers le filtre et l'ouverture.

3. Sac collecteur médico-chirurgical selon la revendication 2, caractérisé en ce que la surface intérieure (53) du filtre (52) est imperméable aux gaz de sorte que le flux de gaz va radialement du bord du filtre vers l'ouverture (54) dans l'élément (62) imperméable aux gaz.

4. Sac collecteur médico-chirurgical selon l'une quelconque des revendications précédentes, caractérisé en ce que le joint comporte une bande flexible adhésive (66).

5. Sac collecteur médico-chirurgical selon la revendication 4, caractérisé en ce que les deux passages d'évents (50, 54 et 60, 64) s'ouvrent en des endroits espacés l'un de l'autre de façon que chacun des passages d'évent peut être découvert en retirant la bande adhésive flexible (66).

6. Procédé d'utilisation d'un sac collecteur médico-chirurgical du type comportant une paroi avant (42) et une paroi arrière (40) en un matériau flexible, la paroi arrière ayant une ouverture (48) dans le sac par laquelle les déjections peuvent entrer dans le sac, des moyens (2, 47) pour fixer le sac au porteur, et un évent (60, 64, 50, 52, 54, 66) dans l'une des parois ayant chacun un passage filtré (50,54) et un passage non filtré (60, 64), le procédé comportant les étapes consistant à ouvrir le passage non filtré (60, 64), à séparer les parois (40 et 42) l'une de l'autre de façon que de l'air entre dans le sac par le passage non filtré et ensuite à fermer l'évent (60, 64, 50, 52, 54, 66) pour retenir de l'air dans le sac et à maintenir ainsi les deux parois séparées l'une de l'autre dans la zone de l'ouverture (49).
